# EUROPÄISCHE PATENTSCHRIFT

(11) **EP 4 414 352 B1**
(45) Veröffentlichungstag und Bekanntmachung des Hinweises auf die Patenterteilung: **27.05.2026**
(21) Anmeldenummer: 23155309.0
(22) Anmeldetag: 07.02.2023
(51) Int. Cl.: C07C 31/04, C07C 29/151

(54) **VERFAHREN ZUR HERSTELLUNG VON METHANOL AUS SYNTHESEGAS MIT HOHEM ANTEIL AN INERTEN GASKOMPONENTEN**
PROCESS FOR THE PRODUCTION OF METHANOL FROM SYNTHESIS GAS CONTAINING A HIGH PROPORTION OF INERT GAS COMPONENTS
PROCÉDÉ DE PRODUCTION DE MÉTHANOL À PARTIR DE GAZ DE SYNTHÈSE CONTENANT UNE TENEUR ÉLEVÉE EN COMPOSANTS DE GAZ INERTES

(43) Veröffentlichungstag der Anmeldung: 14.08.2024
(73) Patentinhaber: L'AIR LIQUIDE, SOCIETE ANONYME POUR L'ETUDE ET L'EXPLOITATION DES PROCEDES GEORGES CLAUDE, 75007 Paris (FR)
(72) Erfinder: Haag, Stéphane, 60388 Frankfurt (DE); Renner, Thomas, 60318 Frankfurt (DE); Castillo-Welter, Frank, 60388 Frankfurt (DE); Do, Nga Thi Quynh, 38116 Braunschweig (DE)
(74) Vertreter: Air Liquide

(56) Entgegenhaltungen:
- WO-A1-2017/021245
- WO-A1-2021/043560
- GB-A- 2 142 331

## Beschreibung

### Technisches Gebiet der Erfindung

Die Erfindung betrifft ein Verfahren zur Herstellung von Methanol aus Synthesegas, insbesondere aus Synthesegas mit einem hohen Anteil an inerten Gaskomponenten.

### Stand der Technik

Methanol wird im industriellen Maßstab aus Synthesegas, einem Gemisch aus Kohlenstoffoxiden und Wasserstoff, hergestellt. Synthesegas wird üblicherweise aus fossilen Einsatzgasen, wie Erdgas, durch Verfahren wie Dampfreformierung (SMR) oder autotherme Reformierung (ATR) erzeugt. Dabei handelt es sich um etablierte industrielle Verfahren, welche ein Synthesegasgemisch erzeugen, welches fast ausschließlich aus Kohlenmonoxid, Kohlendioxid, und Wasserstoff besteht. Inerte Bestandteile wie Methan oder Stickstoff machen dabei nur einen geringfügigen Anteil im Synthesegasgemisch aus.

Methanol kann auf Basis kohlendioxidreicher Einsatzgase hergestellt werden, unter Einsatz hoher Recycle-Raten oder bei mehrstufigen Synthesen mit Zwischenkondensation des Rohmethanols auch aus praktisch Kohlenmonoxid-freien Einsatzgasen. In letzterem Fall ist das eigentliche zu Methanol umgesetzte Synthesegas somit eine Mischung aus Kohlendioxid und Wasserstoff.

Um industriell erzeugte Kohlendioxid-Emissionen zu reduzieren, kann Kohlendioxid entweder abgeschieden und sequestriert werden (carbon capture and storage, CCS), oder nach der Abscheidung einer weiteren Verwertung (carbon capture and utilization, CCU) zugeführt werden. Im Falle von CCU bietet sich dabei die Methanol-synthese auf Basis kohlendioxidreicher Einsatzgase an, wie beispielsweise auf der Basis von industriellen Abgasen. Zumindest können solche Einsatzgase "konventionellem" Synthesegas beigemischt werden, da sie als solche auch nach Hinzufügung von Wasserstoff nicht immer unmittelbar für die Methanolsynthese geeignet sind.

Die vorgenannten Einsatzgase weisen jedoch in der Regel einen hohen Anteil an inerten Bestandteilen auf, genauer gesagt einen hohen Anteil an Gaskomponenten, welche unter Methanolsynthese-Bedingungen inert sind, das heißt nicht reagieren, und damit nicht zur Produktbildung beitragen. Bei der Anwendung hoher Recycle-Raten bei konventionellen einstufigen Methanolsynthesen stellt dies ein Problem dar, da sich inerte Bestandteile am Reaktoreinlass sammeln, was die Reaktorlast erheblich erhöht, einen hohen Druckabfall über den Reaktor verursacht, und die Ausbeute in Bezug auf die Kohlenstoffumwandlung verringert. Schließlich steigt dadurch auch die Last des Synthesegas- und des Recyclegas-Kompressors, was zu höheren Betriebskosten (opex) führt. Auch die Querschnitte der Rohrleitungen müssen entsprechend groß dimensioniert werden, was die Investitionskosten (capex) steigert.

Die vorgenannten Probleme treffen teilweise auch auf mehrstufige Methanolsynthesen mit Zwischenkondensation des Rohmethanols zu. Diese sind üblicherweise so ausgelegt, dass nahezu der gesamte Kohlenstoff des Synthesegases in einem einzigen Durchgang durch alle Reaktorstufen zu Methanol umgesetzt wird. Dadurch ist eine Rückführung des nicht umgesetzten Synthesegases in vielen Fällen nicht erforderlich.

Um Einsatzgase mit hohen Anteilen an inerten Bestandteilen für eine Methanolsynthese verwerten zu können, ist aus vorgenannten Gründen in der Regel eine Reinigung des Einsatzgases, das heißt eine aufwändige Abtrennung der inerten Bestandteile, erforderlich, bevor diese der Synthese zugänglich gemacht werden können.

GB 2 142 331 A offenbart die Herstellung von Methanol, bei der aus dem Methanol-synthese-Kreislauf ein Spülgas ausgeschleust wird, aus welchem Wasserstoff mit Hilfe einer Druckwechseladsorptionsvorrichtung abgetrennt wird. Der so gewonnene Wasserstoffstoffstrom wird über einen Expander adiabatisch druckentspannt, und die so gewonnene Energie wird zum Antrieb einen adiabatischen Verdichters genutzt.

WO 2017/021245 A1 offenbart ein Verfahren zur chemischen Synthese von Methanol aus verdichteten gasförmigen Edukten. Aus dem gebildeten Produktgemisch wird durch Druckabsenkung Energie entnommen, welche bei der Kompression der Edukte benutzt wird. Dies erfolgt vorzugsweise mittels gasförmiger Teile des Produktgemischs.

WO 2021/043560 A1 offenbart die katalytische Umsetzung eines Synthesegasstroms zu einem Alkane, Alkene und Alkohole enthaltenden Produktstrom. Der unter erhöhtem Druck stehende Produktstrom wird in einer Turbine entspannt, wobei elektrische Energie für den Strombedarf des Prozesses gewonnen wird.

### Beschreibung der Erfindung

In vielen Fällen muss davon ausgegangen werden, dass sich eine aufwändige Abtrennung von inerten Gasbestandteilen aus dem jeweiligen Ausgangsstoff aus wirtschaftlichen Gründen nicht lohnt. Es muss daher ein anderer Weg gefunden werden, wie eine entsprechende Methanolproduktion auch bei hohem Anteil an inerten Bestandteilen im Einsatzgas technisch und wirtschaftlich sinnvoll betrieben werden kann.

Eine zentrale Rolle spielt dabei der Synthesegaskompressor, das heißt derjenige Kompressor, der das Synthesegasgemisch auf den für die Methanolsynthese erforderlichen Synthesedruck verdichtet. Dabei handelt es sich in der Regel um eine Maschine, welche mit einem hohen Aufwand an elektrischer Energie betrieben wird.

Ziel sollte sein, die Menge an bereitzustellender extern erzeugter elektrischer Energie für den Synthesegaskompressor auch beim Betrieb mit einem Einsatzgas mit hohem Anteil inerter Bestandteile zu verringern oder gleich zu halten, gegenüber dem Betrieb des Synthesegaskompressors mit einem Einsatzgas ohne hohen Anteil inerter Bestanteil. Ein solches Ergebnis kann beispielsweise durch eine verbesserte Prozessintegration erreicht werden, indem beispielsweise prozessintern erzeugte Energie so genutzt wird, dass diese den Aufwand für die Bereitstellung prozessextern erzeugter Energie verringert.

EP 2 228 357 A1 offenbart ein Verfahren zur Herstellung von Methanol, in dem ein Purge-Gasstrom mit Hilfe eines Expanders druckentspannt wird. Dabei erzeugte mechanische Arbeit wird prozessintern genutzt, beispielsweise für den Antrieb eines Recycle-Gas Kompressors.

Nachteilig daran ist, dass der Purge-Gasstrom innerhalb einer Methanolsynthese mit Rückführung des nicht umgesetzten Synthesegases nur einen relativ geringen Teilvolumenstrom gegenüber dem Gesamtvolumenstrom des im Reaktor nicht umgesetzten Restgases ausmacht, und die Rückgewinnung von Energie entsprechend auf diesen Teilvolumenstrom limitiert ist.

Bei der Verwendung von Einsatzgasen mit hohem Anteil an inerten Gaskomponenten wäre es jedoch wünschenswert, einen möglichst großen Anteil eines Gasstroms für die prozessinterne Energierückgewinnung nutzen zu können, oder zusätzliche Möglichkeiten für die prozessinterne Energierückgewinnung zu generieren. Dabei besteht die Aufgabe in jedem Fall darin, den Energieeinsatz für den SynthesegasKompressor zu minimieren.

Eine Aufgabe der vorliegenden Erfindung besteht somit darin, die vorgenannten Nachteile des Standes der Technik zumindest teilweise zu überwinden.

Insbesondere besteht eine Aufgabe der vorliegenden Erfindung darin, die Möglichkeiten der prozessinternen Nutzung von Energie zu erweitern oder zu verbessern.

Weiterhin besteht eine Aufgabe der vorliegenden Erfindung darin, die Prozessintegration der Methanolherstellung in Bezug auf den externen Energiebedarf weiter zu verbessern, so dass sich die Methanolherstellung auch mit Einsatzgasen technisch und wirtschaftlich sinnvoll realisieren lässt, welche einen hohen Anteil an inerten Bestandteilen aufweisen, ohne dass eine vorherige Abtrennung der inerten Bestandteile aus dem jeweiligen Einsatzgas erfolgen muss.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Gemäß einem ersten Aspekt der Erfindung wird ein Verfahren zur Herstellung von Methanol vorgeschlagen, aufweisend die Verfahrensschritte
a) Bereitstellen eines Synthesegasstroms, aufweisend zumindest ein Kohlenstoffoxid, Wasserstoff, und eine inerte Gaskomponente;
b) Verdichten des Synthesegasstroms auf Synthesedruck in einer Synthesegas-Verdichtungsvorrichtung;
c) Umsetzen des verdichteten Synthesegasstroms an einem Methanol-synthese-Katalysator zu einem Produktstrom, wobei der Produktstrom zumindest Methanol, Wasser, nicht umgesetztes Synthesegas und die inerte Gaskomponente aufweist, wobei das Umsetzen des verdichteten Synthesegasstroms in einer Reaktoranordnung erfolgt, wobei die Reaktoranordnung zumindest eine Reaktorstufe und eine Phasentrennvorrichtung aufweist;
d) Auftrennen des Produktstroms in der Phasentrennvorrichtung in einen flüssigen Rohmethanolstrom, aufweisend zumindest Methanol und Wasser, sowie einen Restgasstrom, aufweisend zumindest nicht umgesetztes Synthesegas und die inerte Gaskomponente;
e) Entspannen zumindest eines Teils des Rohmethanolstroms in einer Flüssigkeit-Entspannungsvorrichtung auf einen Entspannungsdruck, welcher niedriger ist als der Synthesedruck, wobei die Flüssigkeit-Entspannungsvorrichtung mechanische Arbeit verrichtet;
f) Übertragen zumindest eines Teils der von der Flüssigkeit-Entspannungsvorrichtung verrichteten mechanischen Arbeit auf die Synthesegas-Verdichtungsvorrichtung, zum Antrieb der Synthesegas-Verdichtungsvorrichtung, oder
   Umwandeln zumindest eines Teils der von der Flüssigkeit-Entspannungsvorrichtung verrichteten mechanischen Arbeit in elektrische Energie, und Nutzung der elektrischen Energie zum Antrieb der Synthesegas-Verdichtungsvorrichtung.

Eine Reaktorstufe der Reaktoranordnung weist den Methanolsynthese-Katalysator auf. Dabei kann es sich um jeden dem Fachmann bekannten für die Methanolsynthese geeigneten Katalysator handeln, beispielsweise um einen Katalysator auf Kupfer-Basis. Der Katalysator kann beispielsweise als Festbett in Form von Pellets oder als strukturierte Packung ausgestaltet sein.

Eine Phasentrennvorrichtung ist zum Abtrennen des Produktstroms vom Restgasstrom konfiguriert. Gemäß einem Beispiel weißt die Phasentrennvorrichtung eine Kühlstufe in Form eines Wärmeaustauschers zur Kondensation des Produktstrom auf, sowie eine Trennstufe zur Abtrennung der kondensierten (flüssigen) Phase von der Gasphase auf.

Erfindungsgemäß wird zumindest ein Teil des Rohmethanolstroms in einer Flüssigkeit-Entspannungsvorrichtung auf einen Entspannungsdruck entspannt, und die von der Entspannungsvorrichtung dabei geleistete mechanische Arbeit wird prozessintern genutzt. Die Nutzung kann dabei unmittelbar erfolgen, durch mechanischen Antrieb der Synthesegas-Verdichtungsvorrichtung, oder mittelbar, indem die von der Entspannungsvorrichtung geleistete mechanische Arbeit zunächst in elektrische Energie umgewandelt wird. Diese wird anschließend zum Antrieb der Synthesegas-Verdichtungsvorrichtung genutzt.

Optional kann der Restgasstrom in einer Restgas-Verdichtungsvorrichtung auf Synthesedruck rückverdichtet und mit dem verdichteten Restgasstrom zusammengeführt werden. Gemäß dieser optionalen Ausführungsform wird der Restgasstrom somit wieder zum Einlass der Reaktorstufe zurückgeführt und mit dem verdichteten Synthesegas zum Rohmethanolstrom umgesetzt. Optional kann die von der Flüssigkeit-Entspannungsvorrichtung verrichtete mechanische Arbeit auch auf die Restgas-Verdichtungsvorrichtung, zum Antrieb der Restgas-Verdichtungsvorrichtung, genutzt werden, oder die von der Flüssigkeit-Entspannungsvorrichtung verrichtete mechanische Arbeit wird in elektrische Energie umgewandelt, und die elektrische Energie zum Antrieb der Restgas-Verdichtungsvorrichtung genutzt.

Bei der Flüssigkeit-Entspannungsvorrichtung handelt es sich beispielsweise um eine Entspannungsmaschine, die nach dem Verdrängerprinzip (zum Beispiel Kolbenentspannungsmaschine), oder dem Strömungsprinzip (zum Beispiel Turboentspannungsmaschine, Entspannungsturbine) arbeitet. Die Auswahl der Flüssigkeit-Entspannungsvorrichtung richtet sich nach dem zu entspannenden Volumenstrom des Rohmethanolstroms und den vorherrschenden Drücken.

Der Entspannungsdruck ist vorzugsweise um einen Faktor von mindestens 5, oder von mindestens 10, oder von mindestens 15, oder von mindestens 20, oder von mindestens 25, oder von mindestens 50 niedriger als der Synthesedruck. Gemäß einem Beispiel liegt der Synthesedruck in einem Bereich von 60 bar bis 90 bar, und der Entspannungsdruck liegt in einem Bereich von Atmosphärendruck (ca. 1 bar absolut) bis 5 bar.

Wird die von der Flüssigkeit-Entspannungsvorrichtung geleistete Arbeit zunächst in elektrische Energie umgewandelt, so kann dies beispielsweise durch einen Generator erfolgen. Der dadurch erzeugte elektrische Strom kann anschließend beispielsweise einen Elektromotor antreiben, welcher die Synthesegas-Verdichtungsvorrichtung antreibt.

Bei der Synthesegas-Verdichtungsvorrichtung handelt es sich um einen oder mehrere parallel und/oder seriell geschaltete Kompressoren (Verdichter), der/die wiederum entweder nach dem Verdrängerprinzip (zum Beispiel Kolbenkompressor) oder nach dem Strömungsprinzip (zum Beispiel Turbokompressor) arbeitet/arbeiten.

Der Synthesegasstrom weist zumindest ein Kohlenstoffoxid, Wasserstoff, und eine inerte Gaskomponente auf. Unter einem "Kohlenstoffoxid" wird dabei Kohlenmonoxid (CO) oder Kohlendioxid (CO₂) verstanden. Bei der inerten Gaskomponenten kann es sich stofflich betrachtet um ein einziges Gas (zum Beispiel ausschließlich Stickstoff), oder eine Mischung von Gasen (zum Beispiel Stickstoff, Argon und Methan) handeln. Unter "inert" ist dabei zu verstehen, dass die Gaskomponente unter den Bedingungen der Methanolsynthese nicht reagiert, also weder zum Produkt, noch zu einem unerwünschten Nebenprodukt, umgesetzt wird.

Gemäß einer Ausführungsform, insbesondere bei einer Methanolsynthese mit nur einer oder mit zwei Reaktorstufen, wird zumindest ein Teil des Restgasstroms zum Eingang der oder einer der Reaktorstufe(n) zurückgeführt. Von einem Teil dieses Rückführstroms kann ein Purge-Strom abgezweigt werden, um die Kumulation der inerten Bestandteile in der Syntheseschleife zu verhindern. Der Purge-Strom kann mit Hilfe einer Gas-Entspannungsvorrichtung, wie aus EP 2 228 357 A1 bekannt, zusätzlich zum Verrichten mechanischer Arbeit und damit zur prozessinternen Energieerzeugung genutzt werden.

Eine Ausführungsform des Verfahrens ist dadurch gekennzeichnet, dass die Reaktoranordnung eine Mehrzahl n in Serie angeordneter Reaktorstufen und eine Mehrzahl p Phasentrennvorrichtungen aufweist, wobei jeder der Reaktorstufen eine Phasentrennvorrichtung zugeordnet ist, wobei die Phasentrennvorrichtung jeweils stromabwärts zur zugeordneten Reaktorstufe angeordnet ist, und in jeder der Phasentrennvorrichtungen ein flüssiger Rohmethanolteilstrom und ein Restgasteilstrom erzeugt wird, und wobei der in einer Phasentrennvorrichtung erzeugte Restgasteilstrom zumindest teilweise in die jeweils nachfolgend angeordnete Reaktorstufe eingeschleust wird, und wobei der in der letzten Phasentrennvorrichtung erzeugte Restgasteilstrom aus der Reaktoranordnung ausgeschleust wird.

Gemäß dieser Ausführungsform weist die Reaktoranordnung mindestens zwei Reaktorstufen und mindestens zwei Phasentrennvorrichtungen auf. Jeder Reaktorstufe ist eine Phasentrennvorrichtung zugeordnet, welche stromabwärts zur jeweiligen Reaktorstufe angeordnet ist. Der aus einer Reaktorstufe ausgeschleuste Produktteilstrom wird in der stromabwärts angeordneten Phasentrennstufe in einen Rohmethanolteilstrom und einen Restgasteilstrom aufgetrennt. Der Rohmethanolteilstrom wird als Rohproduktteilstrom aus der Reaktoranordnung ausgeschleust und einer weiteren Aufarbeitung unterzogen. Der aus einer Phasentrennstufe abgezogene Restgasteilstrom wird vorzugsweise vollständig in die jeweils nachfolgende der in Serie angeordneten Reaktorstufen eingeschleust. Es kann auch nur ein Teil in die jeweils nachfolgende Reaktorstufe eingeschleust werden, und der übrige Teil wird einer anderen Verwendung unterzogen.

Eine Ausnahme bildet dabei die letzte der in Serie angeordneten Phasentrennstufen. Der aus dieser letzten Phasentrennstufe ausgeschleuste Restgasteilstrom wird aus der Reaktoranordnung ausgeschleust und einer weiteren Verwendung unterzogen.

Die Konfiguration der Reaktorstufen und der Phasentrennvorrichtungen innerhalb der Reaktoranordnung gemäß dieser Ausführungsform kann auch als Multistufen-Reaktoranordnung mit Zwischenkondensation bezeichnet werden.

Eine Ausführungsform des Verfahrens ist diesbezüglich dadurch gekennzeichnet, dass das Verfahren die weiteren Verfahrensschritte aufweist,
g) Entspannen zumindest eines Teils des aus der Reaktoranordnung ausgeschleusten Restgasteilstroms in einer Gas-Entspannungsvorrichtung auf einen Entspannungsdruck, welcher niedriger ist als der Synthesedruck, wobei die Gas-Entspannungsvorrichtung mechanische Arbeit verrichtet;
h) Übertragen zumindest eines Teils der von der Gas-Entspannungsvorrichtung verrichteten mechanischen Arbeit auf die Synthesegas-Verdichtungsvorrichtung, zum Antrieb der Synthesegas-Verdichtungsvorrichtung, oder
   Umwandeln zumindest eines Teils der von der Gas-Entspannungsvorrichtung verrichteten mechanischen Arbeit in elektrische Energie, und Nutzung der elektrischen Energie zum Antrieb der Synthesegas-Verdichtungsvorrichtung.

Diese Ausführungsform bezieht sich auf die Konfiguration der Reaktoranordnung als Multistufen-Reaktoranordnung. In vorteilhafter Weise wird gemäß dieser Ausführungsform auch der aus der Reaktoranordnung ausgeschleuste Restgasteilstrom zur Verrichtung mechanischer Arbeit genutzt, indem dieser Restgasteilstrom in einer Gas-Entspannungsvorrichtung auf einen Entspannungsdruck entspannt wird.

Bei der Verwendung einer Anzahl Reaktorstufen n, welche dazu führt, dass über alle Reaktorstufen ein vollständiger oder nahezu vollständiger Kohlenstoffumsatz erzielt wird, enthält der aus der letzten Phasentrennstufe ausgeschleuste Restgasteilstrom praktisch keine Wertgase mehr, sondern besteht im Wesentlichen aus den Gasen der inerten Gaskomponente. Daher ist es insbesondere sinnvoll, den Restgasteilstrom für die prozessinterne Gewinnung von mechanischer oder elektrischer Energie für den Antrieb des Synthesegaskompressors zu nutzen. Dies ist insbesondere in Verbindung mit einem Synthesegasstrom sinnvoll, welcher eine inerte Gaskomponenten mit einem Anteil von mindestens 1 Vol.-% im Synthesegas aufweist.

Im Gegensatz zu dem aus EP 2 228 357 A1 bekannten Verfahren wird nicht aus dem Restgasstrom ein Anteil als Purge-Strom abgezweigt, sondern der vollständige Restgasstrom kann zur prozessinternen Gewinnung von mechanischer oder elektrischer Energie genutzt werden.

Der Entspannungsdruck des entspannten Restgasteilstroms ist vorzugsweise um einen Faktor von mindestens 5, oder von mindestens 10, oder von mindestens 15, oder von mindestens 20, oder von mindestens 25, oder von mindestens 50 niedriger als der Synthesedruck. Gemäß einem Beispiel liegt der Synthesedruck in einem Bereich von 60 bar bis 90 bar, und der Entspannungsdruck liegt in einem Bereich von Atmosphärendruck (ca. 1 bar absolut) bis 5 bar.

Bei der Gas-Entspannungsvorrichtung handelt es sich beispielsweise um eine Entspannungsmaschine, die nach dem Verdrängerprinzip (zum Beispiel Kolbenentspannungsmaschine), oder dem Strömungsprinzip (zum Beispiel Turboentspannungsmaschine, Entspannungsturbine) arbeitet. Die Auswahl der Gas-Entspannungsvorrichtung richtet sich nach dem zu entspannenden Volumenstrom des Rohmethanolstroms und den vorherrschenden Drücken.

Je nach Zusammensetzung des Restgasteilstroms kann es sinnvoll sein, den druckentspannten Restgasteilstrom einer Wasserstoff-Rückgewinnungsvorrichtung zuzuführen und den dabei rückgewonnen Wasserstoff zur Methanolsynthese zurückzuführen.

Eine Ausführungsform des Verfahrens ist dabei dadurch gekennzeichnet, dass der gesamte aus der Reaktoranordnung ausgeschleuste Restgasteilstrom in der Gas-Entspannungsvorrichtung entspannt wird.

Die Nutzung des gesamten aus der Reaktoranordnung ausgeschleusten Restgasteilstroms für die prozessinterne Gewinnung von mechanischer oder elektrischer Energie ist insbesondere dann sinnvoll, wenn die Anzahl n Reaktorstufen so hoch ist, dass über alle Reaktorstufen n der Reaktoranordnung ein vollständiger oder im Wesentlichen vollständiger Kohlenstoffumsatz erzielt wird.

Alternativ dazu ist auch möglich, dass ein Anteil des aus der Reaktoranordnung ausgeschleusten Restgasteilstroms in der Gas-Entspannungsvorrichtung entspannt wird, und ein weiterer Anteil des aus der Reaktoranordnung ausgeschleusten Restgasteilstroms in einer Restgas-Verdichtungsvorrichtung auf Synthesedruck verdichtet wird, und der verdichtete Restgasteilstrom zumindest zu einer der Mehrzahl in Serie angeordneter Reaktorstufen zurückgeführt wird, vorzugsweise zur ersten der Mehrzahl in Serie angeordneter Reaktorstufen zurückgeführt wird.

Dies ist insbesondere dann sinnvoll, wenn der aus der letzten Phasentrennvorrichtung ausgeschleuste Restgasteilstrom noch einen verwertbaren Anteil Kohlenstoffoxide und Wasserstoff enthält. In diesem Fall wird nur ein Teil des Restgasteilstroms für die prozessinterne Gewinnung von mechanischer oder elektrischer Energie genutzt, der andere Teil wird auf Synthesedruck rückverdichtet und vorzugsweise zur ersten der Mehrzahl in Serie angeordneter Reaktorstufen zurückgeführt.

Die prozessintern durch die Entspannung des Restgasteilstroms gewonnene mechanische oder elektrische Energie kann optional auch zum Antrieb der Restgas-Verdichtungsvorrichtung genutzt werden.

Gemäß einer Ausführungsform wird ein Teil des auf Entspannungsdruck entspannten Restgasteilstroms in der Synthesegas-Verdichtungsvorrichtung auf Synthesedruck rückverdichtet, und der verdichtete Restgasteilstrom wird der Reaktoranordnung zugeführt.

Bei entsprechender Zusammensetzung des Restgasteilstroms ist es sinnvoll, diesen nach der Druckentspannung zur Synthesegas-Verdichtungsvorrichtung zurückzuführen. Hier wird der Restgasteilstrom mit dem Synthesegasstrom zusammen auf Synthesedruck verdichtet und anschließend der Reaktoranordnung zugeführt.

Eine Ausführungsform des Verfahrens ist dadurch gekennzeichnet, dass der Anteil der inerten Gaskomponente im Synthesegasstrom zumindest 1 Vol.-% beträgt, oder zumindest 5 Vol.-% beträgt, oder zumindest 10 Vol.-% beträgt, oder zumindest 20 Vol.-% beträgt.

Insbesondere liegt der Anteil der inerten Gaskomponente im Synthesegasstrom bei 0,5 Vol.-% bis 30 Vol.-%, oder bei 1 Vol.-% bis 20 Vol.-%, oder bei 1 Vol.-% bis 10 Vol.-%.

Eine Ausführungsform des Verfahrens ist dadurch gekennzeichnet, dass die inerte Gaskomponente Stickstoff und/oder Methan aufweist.

Eine Ausführungsform des Verfahrens ist dadurch gekennzeichnet, dass der Synthesedruck mindestens 70 bar beträgt, oder mindestens 75 bar beträgt, oder mindestens 80 bar beträgt, oder mindestens 85 bar beträgt.

Eine Ausführungsform des Verfahrens ist dadurch gekennzeichnet, dass der Rohmethanolstrom in der Flüssigkeit-Entspannungsvorrichtung auf einen Druck von 1 bar bis 5 bar entspannt wird, vorzugsweise auf einen Druck von 1 bar bis 3 bar entspannt wird. Gemäß einer bevorzugten Ausführungsform wird der Rohmethanolstrom in der Flüssigkeit-Entspannungsvorrichtung auf einen Druck entspannt, welcher Atmosphärendruck entspricht, also in etwa 1 bar (absolut) entspricht.

Eine besonders bevorzugte Ausführungsform des Verfahrens ist dadurch gekennzeichnet, dass der Rohmethanolstrom einer stromabwärts angeordneten thermischen Trennvorrichtung zur Trennung des Rohmethanols in Methanol und Wasser zugeführt wird, wobei die thermische Trennvorrichtung bei einem vorgegebenen Druck betrieben wird, und der Rohmethanolstrom in der Flüssigkeit-Entspannungsvorrichtung auf einen Druck entspannt wird, welcher dem vorgegebenen Druck in der thermischen Trennvorrichtung entspricht.

Insbesondere wird der Rohmethanolstrom in der Flüssigkeit-Entspannungsvorrichtung auf einen Druck entspannt, welcher im Wesentlichen dem vorgegebenen Druck in der thermischen Trennvorrichtung entspricht.

Durch diese Maßnahme kann der druckentspannte Rohmethanolstrom anschließend unmittelbar in die stromabwärts angeordnete thermische Trennvorrichtung eingespeist werden.

Gemäß einer Ausführungsform ist der thermischen Trennvorrichtung eine Niederdruck-Phasentrennvorrichtung vorgeschaltet. Gemäß dieser Konfiguration ist die der Reaktorstufe unmittelbar nachgeschaltete Phasentrennvorrichtung als Hochdruck-Phasentrennvorrichtung konfiguriert oder sind die den Reaktorstufen nachgeschalteten Phasentrennvorrichtungen als Hochdruck-Phasentrennvorrichtungen konfiguriert. Die Flüssigkeit-Entspannungsvorrichtung ist dann zwischen der Hochdruck-Phasentrennvorrichtung und der Niederdruck-Phasentrennvorrichtung angeordnet, oder zwischen den Hochdruck-Phasentrennvorrichtungen und der Niederdruck-Phasentrennvorrichtung angeordnet.

Bei der thermischen Trennvorrichtung handelt es sich gemäß einem Beispiel um eine Rektifikationskolonne, oder um eine Anordnung mehrerer Rektifikationskolonnen.

Eine Ausführungsform des Verfahrens ist dadurch gekennzeichnet, dass der Restgasteilstrom in der Flüssigkeit-Entspannungsvorrichtung auf einen Druck von 1 bar bis 5 bar entspannt wird, vorzugsweise auf einen Druck von 1 bar bis 3 bar entspannt wird. Gemäß einer bevorzugten Ausführungsform wird der Restgasteilstrom in der Flüssigkeit-Entspannungsvorrichtung auf einen Druck entspannt, welcher Atmosphärendruck entspricht, also in etwa 1 bar (absolut) entspricht.

Eine Ausführungsform des Verfahrens ist dadurch gekennzeichnet, dass für die Mehrzahl n in Serie angeordneter Reaktorstufen und die Mehrzahl p Phasentrennvorrichtungen gilt
- n = 3 bis 6, vorzugsweise n = 4, sowie
- p = 3 bis 6, vorzugsweise p = 4, und wobei gilt
- n = p.

Gemäß einem zweiten Aspekt der Erfindung wird ein Verfahren zur Herstellung von Methanol vorgeschlagen, aufweisend die Verfahrensschritte
a) Bereitstellen eines Synthesegasstroms, aufweisend zumindest ein Kohlenstoffoxid, Wasserstoff, und eine inerte Gaskomponente;
b) Verdichten des Synthesegasstroms auf Synthesedruck in einer Synthesegas-Verdichtungsvorrichtung;
c) Umsetzen des verdichteten Synthesegasstroms an einem Methanol-synthese-Katalysator zu einem Produktstrom, wobei der Produktstrom zumindest Methanol, Wasser, nicht umgesetztes Synthesegas und die inerte Gaskomponente aufweist, wobei das Umsetzen des verdichteten Synthesegasstroms in einer Reaktoranordnung erfolgt, wobei die Reaktoranordnung zumindest eine Reaktorstufe und eine Phasentrennvorrichtung aufweist;
d) Auftrennen des Produktstroms in der Phasentrennvorrichtung in einen flüssigen Rohmethanolstrom, aufweisend zumindest Methanol und Wasser, sowie einen Restgasstrom, aufweisend zumindest nicht umgesetztes Synthesegas und die inerte Gaskomponente;
   wobei die Reaktoranordnung eine Mehrzahl n in Serie angeordneter Reaktorstufen und eine Mehrzahl p Phasentrennvorrichtungen aufweist, wobei jeder der Reaktorstufen eine Phasentrennvorrichtung zugeordnet ist, wobei die Phasentrennvorrichtung jeweils stromabwärts zur zugeordneten Reaktorstufe angeordnet ist, und in jeder der Phasentrennvorrichtungen ein flüssiger Rohmethanolteilstrom und ein Restgasteilstrom erzeugt wird, und wobei der in einer Phasentrennvorrichtung erzeugte Restgasteilstrom zumindest teilweise in die jeweils nachfolgend angeordnete Reaktorstufe eingeschleust wird, und wobei der in der letzten Phasentrennvorrichtung erzeugte Restgasteilstrom aus der Reaktoranordnung ausgeschleust wird, und
   weiterhin aufweisend die Verfahrensschritte
e) Entspannen zumindest eines Teils des aus der Reaktoranordnung ausgeschleusten Restgasteilstroms in einer Gas-Entspannungsvorrichtung auf einen Entspannungsdruck, welcher niedriger ist als der Synthesedruck, wobei die Gas-Entspannungsvorrichtung mechanische Arbeit verrichtet;
f) Übertragen zumindest eines Teils der von der Gas-Entspannungsvorrichtung verrichteten mechanischen Arbeit auf die Synthesegas-Verdichtungsvorrichtung, zum Antrieb der Synthesegas-Verdichtungsvorrichtung, oder
   Umwandeln zumindest eines Teils der von der Gas-Entspannungsvorrichtung verrichteten mechanischen Arbeit in elektrische Energie, und Nutzung der elektrischen Energie zum Antrieb der Synthesegas-Verdichtungsvorrichtung.

In vorteilhafter Weise wird gemäß dem zweiten Aspekt des Verfahrens der aus der Reaktoranordnung ausgeschleuste Restgasteilstrom zur Verrichtung mechanischer Arbeit genutzt, indem dieser Restgasteilstrom in einer Gas-Entspannungsvorrichtung auf einen Entspannungsdruck entspannt wird.

Bei der Verwendung einer Anzahl Reaktorstufen n, welche dazu führt, dass über alle Reaktorstufen ein vollständiger oder nahezu vollständiger Kohlenstoffumsatz erzielt wird, enthält der aus der letzten Phasentrennstufe ausgeschleuste Restgasteilstrom praktisch keine Wertgase mehr, sondern besteht im Wesentlichen aus den Gasen der inerten Gaskomponente. Daher ist es insbesondere sinnvoll, den Restgasteilstrom für die prozessinterne Gewinnung von mechanischer oder elektrischer Energie für den Antrieb des Synthesegaskompressors zu nutzen. Dies ist insbesondere in Verbindung mit einem Synthesegasstrom sinnvoll, welcher eine inerte Gaskomponenten mit einem Anteil von mindestens 1 Vol.-% im Synthesegas aufweist.

Im Gegensatz zu dem aus EP 2 228 357 A1 bekannten Verfahren wird nicht aus dem Restgasstrom ein Anteil als Purge-Strom abgezweigt, sondern der vollständige Restgasstrom kann zur prozessinternen Gewinnung von mechanischer oder elektrischer Energie genutzt werden.

Gemäß einer Ausführungsform des zweiten Aspekts des Verfahrens wird der gesamte aus der Reaktoranordnung ausgeschleuste Restgasteilstrom in der Gas-Entspannungsvorrichtung entspannt.

Gemäß einer Ausführungsform des zweiten Aspekts des Verfahrens wird ein Anteil des aus der Reaktoranordnung ausgeschleusten Restgasteilstroms in der Gas-Entspannungsvorrichtung entspannt, und ein weiterer Anteil des aus der Reaktoranordnung ausgeschleusten Restgasteilstroms wird in einer Restgas-Verdichtungsvorrichtung auf Synthesedruck verdichtet, und der verdichtete Restgasteilstrom wird zumindest zu einer der Mehrzahl in Serie angeordneter Reaktorstufen zurückgeführt, vorzugsweise zur ersten der Mehrzahl in Serie angeordneter Reaktorstufen zurückgeführt.

Gemäß einer Ausführungsform des zweiten Aspekts des Verfahrens wird ein Teil des auf Entspannungsdruck entspannten Restgasteilstroms in der Synthesegas-Verdichtungsvorrichtung auf Synthesedruck rückverdichtet, und der verdichtete Restgasteilstrom wird der Reaktoranordnung zugeführt.

Gemäß einer Ausführungsform des zweiten Aspekts des Verfahrens beträgt der Anteil der inerten Gaskomponente im Synthesegasstrom zumindest 1 Vol.-%, oder zumindest 5 Vol.-%, oder zumindest 10 Vol.-%, oder zumindest 20 Vol.-%.

Gemäß einer Ausführungsform des zweiten Aspekts des Verfahrens weist die inerte Gaskomponente Stickstoff und/oder Methan auf.

Gemäß einer Ausführungsform des zweiten Aspekts des Verfahrens beträgt der Synthesedruck mindestens 70 bar, oder mindestens 75 bar, oder mindestens 80 bar, oder mindestens 85 bar.

Gemäß einer Ausführungsform des zweiten Aspekts des Verfahrens wird der Restgasteilstrom in der Gas-Entspannungsvorrichtung auf einen Druck von 1 bar bis 3 bar entspannt.

Gemäß einer Ausführungsform des zweiten Aspekts des Verfahrens gilt für die Mehrzahl n in Serie angeordneter Reaktorstufen und die Mehrzahl p Phasentrennvorrichtungen
- n = 3 bis 6, vorzugsweise n = 4, sowie
- p = 3 bis 6, vorzugsweise p = 4, und
- n = p.

### Ausführungsbeispiel

Die Erfindung wird im Folgenden durch Ausführungsbeispiele näher erläutert. In der folgenden detaillierten Beschreibung wird auf die beigefügten Zeichnungen verwiesen, die einen Teil der Ausführungsbeispiele bilden und in welchen illustrativ spezifische Ausführungsformen der Erfindung dargestellt sind.

In der folgenden Beschreibung und in den Zeichnungen sind gleiche Elemente mit gleichen Bezugszeichen versehen. Gasströme sind in den Figuren als gestrichelte Linien dargestellt, während Flüssigkeitsströme oder zweiphasige Ströme als durchgezogene Linien dargestellt sind. Die Flussrichtung der jeweiligen Ströme ist durch Pfeile angezeigt.

Es zeigen
- Figur 1: ein Verfahren 1 gemäß einem ersten Beispiel der Erfindung mit einer einstufigen Methanol-Synthese,
- Figur 2: ein Verfahren 2 gemäß einem zweiten Beispiel der Erfindung mit einer vierstufigen Methanol-Synthese,
- Figur 3: ein Verfahren 3 gemäß einem dritten Beispiel der Erfindung mit einer vierstufigen Methanol-Synthese, und
- Figur 4: ein Verfahren 4 gemäß einem vierten Beispiel der Erfindung mit einer vierstufigen Methanol-Synthese

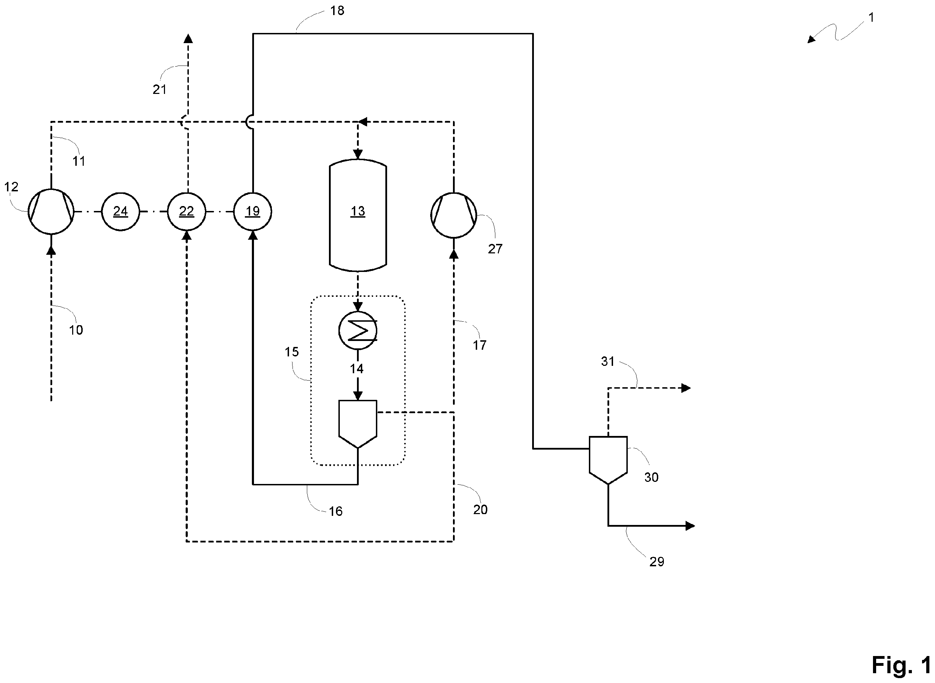

Figur 1 zeigt ein Verfahren 1 gemäß einem ersten Beispiel der Erfindung, in welchem die Synthese des Methanols in einer einstufigen Reaktoranordnung erfolgt.

Ein Synthesegasstrom 10, aufweisend Wasserstoff, Kohlenmonoxid, Kohlendioxid, sowie Stickstoff und Methan als inerte Gaskomponente, wird bereitgestellt und in einer Synthesegas-Verdichtungsvorrichtung 12, hier einem Synthesegaskompressor, auf einen für die Methanolsynthese geeigneten Synthesedruck verdichtet. Der Synthesedruck kann gemäß einem Beispiel 90 bar betragen. Ein resultierender verdichteter Synthesegasstrom 11 wird in eine Reaktorstufe 13 eingeschleust, in welcher der verdichtete Synthesegasstrom 11 zu einem zunächst gasförmigen, und nach Abkühlung zweiphasigen Produktstrom 14 umgesetzt wird, welcher zumindest Methanol, Wasser, sowie nicht umgesetztes Synthesegas (Wasserstoff, Kohlenmonoxid und Kohlendioxid) und die inerte Gaskomponente enthält. Die Reaktorstufe 13 enthält einen geeigneten Methanolsynthese-Katalysator auf Kupferbasis (nicht gezeigt). Da die Reaktion der Methanolbildung aus Synthesegas exotherm verläuft, wird die Reaktorstufe 13 mit Hilfe von siedendem Kesselspeisewasser gekühlt. Der dabei gebildete Dampf kann exportiert werden oder für einen vorgeschalteten Prozess, beispielsweise einen Dampfreformer, verwendet werden. Die Reaktorstufe 13 bildet einen Teil einer Reaktoranordnung, welche zumindest die Reaktorstufe 13 und eine der Reaktorstufe nachgeschaltete Phasentrennvorrichtung 15 aufweist. Die Phasentrennvorrichtung weist zumindest einen Wärmeaustauscher zum Kühlen und Kondensieren des zunächst gasförmigen Rohmethanols zum Produktstrom 14, sowie einen dem Wärmeaustauscher nachgeschalteten Abscheider zum Trennen der flüssigen Rohmethanol-Phase von der gasförmigen Phase auf. Der Wärmeaustauscher und der Abscheider sind nicht mit dedizierten Bezugsziffern versehen.

Aus der Phasentrennvorrichtung 15 wird ein flüssiger Rohmethanolstrom 16 abgezogen, welcher Methanol, Wasser, sowie gegebenenfalls kondensierte Nebenprodukte der Methanolsynthese enthält. Bei dem Abscheider der Phasentrennvorrichtung 15 handelt es sich um einen Hochdruckabscheider, so dass der Rohmethanolstrom 16 einen Druck aufweist, welcher sich hauptsächlich aufgrund unvermeidbarer Druckverluste über die Reaktoranordnung vom Synthesedruck unterscheidet. Der Druck des Rohmethanolstroms 16 ist ausreichend hoch, um für die prozessinterne Erzeugung mechanischer und/oder elektrischer Energie genutzt zu werden. Dafür wird der Rohmethanolstrom 16 einer Flüssigkeit-Entspannungsvorrichtung 19 zugeführt, bei welcher es sich beispielsweise um eine Entspannungsturbine handeln kann. In der Flüssigkeit-Entspannungsvorrichtung 19 wird der Rohmethanolstrom 16 auf einen Druck von circa 2 bar entspannt. Dieser Druck entspricht dem Druck, welcher in einer nachgeschalteten thermischen Trennvorrichtung zur Gewinnung von Reinmethanol (nicht gezeigt) vorherrscht. Der druckentspannte Rohmethanolstrom 18 wird zunächst einem weiteren Abscheider, hier einem Niederdruck-Abscheider 30, zugeführt. Dort findet eine weitere Abtrennung von Gasen statt, welche bei höheren Drücken im Hochdruckabscheider in der flüssigen Phase des Rohmethanols absorbiert bleiben. Die im Niederdruck-Abscheider desorbierten Gase werden als Abgasstrom 31 aus dem Verfahren ausgeleitet. Der von Gasen weiter befreite Rohmethanolstrom 29 wird einer nachfolgenden Destillation zur thermischen Trennung des Rohmethanols in Methanol, Wasser und Nebenprodukte zugeführt (nicht gezeigt).

In der Flüssigkeit-Entspannungsvorrichtung 19 wird mechanische Arbeit verrichtet, welche mit Hilfe eines Generators (nicht gezeigt) in elektrische Energie umgewandelt wird. Dafür steht die Flüssigkeit-Entspannungsvorrichtung 19 in Wirkverbindung mit einem Motor 24, welcher wiederum in Wirkverbindung mit der Synthesegas-Verdichtungsvorrichtung 12 steht, welche über den Motor angetrieben wird. Gemäß dieser Ausführungsform steht die Flüssigkeit-Entspannungsvorrichtung in mittelbarer Wirkverbindung mit der Synthesegas-Verdichtungsvorrichtung 12. Alternativ kann die Flüssigkeit-Entspannungsvorrichtung 19 auch in unmittelbarer Wirkverbindung mit der Synthesegas-Verdichtungsvorrichtung 12 stehen, beispielsweise über eine direkte mechanische Kopplung.

Aus dem Hochdruckabscheider der Phasentrennvorrichtung 15 wird ein Restgasstrom 17 als gasförmige Phase der Abscheidung abgezogen, welcher als Hauptbestandteile nicht umgesetztes Synthesegas (Wasserstoff, Kohlenmonoxid, Kohlendioxid) und die inerte Gaskomponente (Stickstoff, Methan) aufweist. Dieser Restgasstrom 17 wird mit Hilfe einer Restgas-Verdichtungsvorrichtung 27, hier einem Recyclegas-Kompressor, auf Synthesedruck rückverdichtet und mit dem verdichteten Synthesegas-Strom zusammengeführt. Da sich bei kontinuierlicher Rückführung inerte Bestandteile in dem der Reaktorstufe 13 zugeführten Synthesegasstrom konzentrieren würden, wird von dem Restgasstrom 17 kontinuierlich ein Purge-Gasstrom 20 abgezweigt. Dieser Purge-Gasstrom 20 wird einer Gas-Entspannungsvorrichtung 22, hier einem Gas-Expander, zugeführt, in welcher er auf Atmosphärendruck entspannt wird. Der druckentspannte Purge-Gasstrom 21 wird entweder zur Fackel (nicht gezeigt) gesendet oder, falls er einen ausreichend hohen Gehalt an Wertgasen aufweist, einer Wasserstoff-Rückgewinnungsanlage zugeführt (nicht gezeigt). Auch die Gas-Entspannungsvorrichtung 19 steht, wie oben für die Flüssigkeit-Entspannungsvorrichtung 21 ausgeführt, in mittelbarer oder unmittelbarer Wirkverbindung mit der Synthesegas-Verdichtungsvorrichtung 12.

Das folgende Zahlenbeispiel demonstriert auf der Basis von Simulationsdaten den Vorteil des erfindungsgemäßen Verfahrens für eine Konfiguration wie für Figur 1 beschrieben.

In einer Pilotanlage wird Methanol auf der Basis eines stickstoffreichen Synthesegasstroms hergestellt, welcher 12,1 Vol.-% Kohlendioxid, 12,9 Vol.-% Kohlenmonoxid, 49,9 Vol.-% Wasserstoff, und 25,1 Vol.-% Stickstoff enthält. Der Synthesegasstrom wird dem Reaktor mit einem Massenstrom von 8,5 kg/h zugeführt und mit Hilfe des Synthesegaskompressors von zunächst 4 bar auf einen Synthesedruck von 90 bar verdichtet. Es wird ein Rohmethanolstrom mit einem Massenstrom von 3,3 kg/h erhalten, welcher über eine Entspannungsturbine von etwa 90 bar auf 2,1 bar entspannt wird. Mit diesem Druck kann der entspannte Rohmethanolstrom unmittelbar der nachfolgenden Destillation zugeführt werden. Es wird aufgrund der Nutzung der von der Entspannungsturbine verrichteten Arbeit eine Einsparung von 0,011 kW Kompressorleistung erzielt, was einem Anteil von 0,6 % an der Gesamtkompressorleistung entspricht.

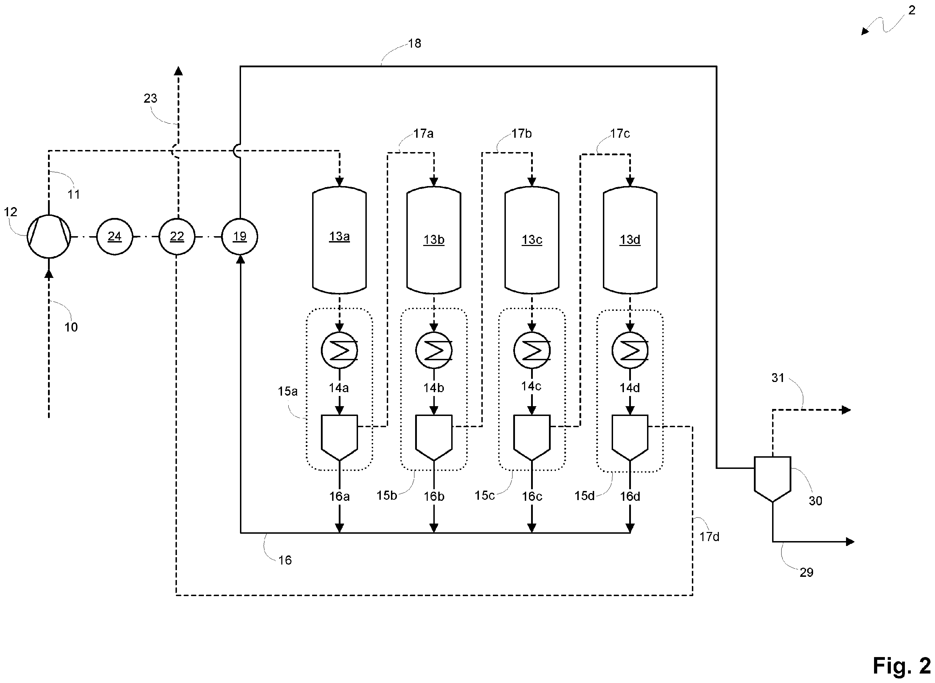

Figur 2 zeigt ein Verfahren 2 gemäß einem zweiten Beispiel der Erfindung, in welchem die Synthese des Methanols in einer vierstufigen Reaktoranordnung erfolgt.

Die Reaktoranordnung gemäß Figur 2 weist vier in Serie geschaltete Reaktorstufen 13a bis 13d auf, wobei jeder Reaktorstufe eine stromabwärts angeordnete Phasentrennvorrichtung zugeordnet ist, wobei die Phasentrennvorrichtungen entsprechend mit 15a bis 15d bezeichnet sind.

Der in Reaktorstufe 13a erzeugte Produktteilstrom 14a wird in der Phasentrennvorrichtung 15a analog des Verfahrens nach Figur 1 mit Hilfe eines Wärmeaustauschers und eines Hochdruckabscheiders in einen Rohmethanolteilstrom 16a als flüssige Phase und einen Restgasteilstrom 17a als gasförmige Phase getrennt. Der Rohmethanolteilstrom 16a wird mit weiteren Rohmethanolteilströmen 16b, 16c und 16d zusammengeführt, woraus der Rohmethanolstrom 16 (oder Rohmethanolgesamtstrom) resultiert. Der aus dem Abscheider abgezogene Restgasteilstrom 17a wird der nachfolgenden Reaktorstufe 13b zugeführt, zur weiteren Umsetzung des verbliebenen Synthesegases des Restgasteilstroms 17a in der zweiten Reaktorstufe 13b. In der der Reaktorstufe 13b zugeordneten und nachgeschalteten Phasentrennvorrichtung 15b wird wiederum ein Rohmethanolteilstrom 16b und ein Restgasteilstrom 17b erzeugt. Analoge Prozesse laufen in den nachfolgenden Reaktorstufen 13c und 13d, sowie den Phasentrennvorrichtungen 15c und 15d ab. Durch die serielle Anordnung der Reaktorstufen 13a bis 13d und der (Zwischen-)Kondensation des Rohmethanols in den Phasentrennvorrichtungen 15a bis 15d kann bei entsprechender Auslegung und Zusammensetzung des Synthesegases über die gesamte Reaktoranordnung ein vollständiger oder zumindest nahezu vollständiger Kohlenstoffumsatz erzielt werden. Der aus der vierten Phasentrennvorrichtung 15d abgezogene Restgasteilstrom 17d enthält daher nur noch geringfügige Mengen an Kohlenstoffoxiden und Wasserstoff, besteht also vor allem aus der inerten Gaskomponente.

Der Rohmethanolstrom 16 wird der Flüssigkeit-Entspannungsvorrichtung 19 zur prozessinternen Gewinnung von mechanischer oder elektrischer Energie zugeführt. Bezüglich der Konfiguration und der Wirkverbindungen der Flüssigkeit-Entspannungsvorrichtung 19, der Gas-Entspannungsvorrichtung 22, des Motors 24, und der Synthesegas-Verdichtungsvorrichtung 12 gelten die Ausführungen wie für Figur 1 beschrieben.

Im Gegensatz zum Verfahren 1 gemäß Figur 1 wird gemäß dem Verfahren 2 der Figur 2 nicht der Purge-Strom, sondern der gesamte Restgasstrom, hier Restgasteilstrom 17d, der Gas-Entspannungsvorrichtung 22 zugeführt. Es resultiert ein druckentspannter Restgasteilstrom 23, welcher beispielsweise zur Fackel gesendet wird (nicht gezeigt).

Das folgende Zahlenbeispiel demonstriert auf der Basis von Simulationsdaten den Vorteil des erfindungsgemäßen Verfahrens für eine Konfiguration wie für Figur 2 beschrieben.

Im industriellen Maßstab wird Methanol auf der Basis eines stickstoffreichen Synthesegasstroms hergestellt, welcher ausschließlich Kohlendioxid als Kohlenoxid-Komponente aufweist. Da der Synthesegasstrom kein Kohlenmonoxid enthält, ist über eine Reaktorstufe mit einem niedrigen Gleichgewichtsumsatz zum Produkt zu rechnen, weshalb eine vierstufige Methanolsynthese mit Zwischenkondensation des Produkts für diese Synthesegaszusammensetzung gewählt wird. Der Synthesegasstrom weist in Bezug auf Kohlendioxid einen Massenstrom von 15240,1 kg/h auf, in Bezug auf Wasserstoff einen Massenstrom von 2095,5 kg/h, und in Bezug auf Stickstoff einen Massenstrom von 294,1 kg/h. Der Synthesegasstrom wird mit Hilfe des Synthesegaskompressors von 10 bar auf einen Druck von 81 bar verdichtet.

Es wird ein Rohmethanolstrom mit einem Massenstrom von 11258 kg/h in Bezug auf den Methanolanteil und einem Massenstrom von 6111 kg/h in Bezug auf den Wasseranteil erhalten. Der Rohmethanolstrom weist darüber hinaus einen Anteil von 328 kg/h gelöstem Kohlendioxid auf, welches in den Hochdruckabscheidern der Phasentrennvorrichtungen 15a bis 15d nicht entfernt wurde.

Der aus der letzten der seriell angeordneten Phasentrennvorrichtungen abgezogene Restgasstrom weist Stickstoff mit einem Massenstrom von 643 kg/h, Kohlendioxid mit einem Massenstrom von 573 kg/h, Methanol mit einem Massenstrom von 78 kg/h, Kohlenmonoxid mit einem Massenstrom von 23 kg/h, Wasserstoff mit einem Massenstrom von 85 kg/h, sowie Wasser mit einem Massenstrom von 3 kg/h auf. Die Hauptkomponenten des Restgasstroms sind somit Stickstoff als inerte Gaskomponente, sowie aufgrund seiner Reaktionsträgheit nicht umgesetztes Kohlendioxid.

Der Rohmethanolstrom wird in einer Entspannungsturbine von 81 bar auf 2,1 bar entspannt, um der nachfolgenden Destillation zugeführt zu werden. Der Restgasteilstrom wird in einem Expander von 81 bar auf 1 bar entspannt.

Der Synthesegaskompressor weist einen Bedarf an elektrischer Energie von 7704 kW auf. Aufgrund der prozessintern genutzten Energie der Entspannungsturbine kann dieser Bedarf um 116,3 kW gesenkt werden, was einer Energieeinsparung von 1,5 % entspricht. Dabei handelt es sich insbesondere im Falle eines Industrieprozesses um signifikante Einsparungen, die langfristig den apparativen Mehraufwand überkompensieren. Gleichzeitig wird im Vergleich mit dem obigen Beispiel klar, dass die erzielte Energieeinsparung proportional zum Maßstab des Prozesses ist (Vergleich Pilotmaßstab und Industriemaßstab).

Weiterhin kann der Bedarf an elektrischer Energie für den Synthesegaskompressor aufgrund der prozessintern genutzten Energie des Expanders zusätzlich um 53,4 kW gesenkt werden, was einer weiteren Energieeinsparung von 0,7 % entspricht. Somit wird eine Gesamteinsparung von 2,2 % an elektrischer Energie erzielt.

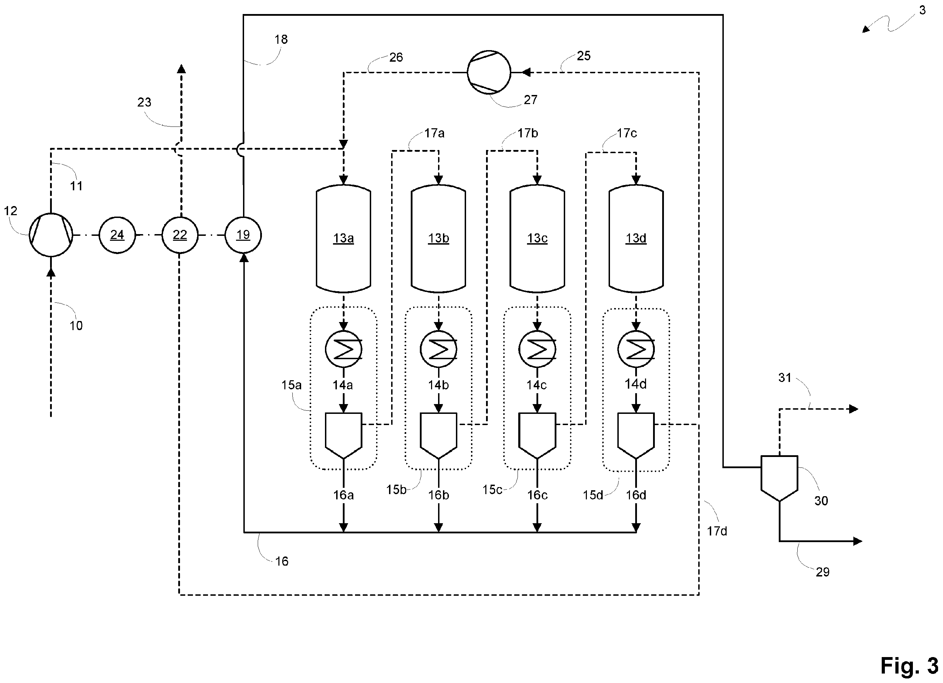

Figur 3 zeigt ein Verfahren 3 gemäß einem dritten Beispiel der Erfindung, in welchem die Synthese des Methanols analog dem Verfahren 2 in einer vierstufigen Reaktoranordnung erfolgt.

Wie im vorherigen Zahlenbeispiel gezeigt, kann der Restgasteilstrom 17d noch Wertgase, das heißt zu Methanol umsetzbare Gase wie Wasserstoff, Kohlenmonoxid und Kohlendioxid, enthalten. Je nach Anteil dieser Wertgase kann es sich lohnen, einen Teil vom Restgasteilstrom 17d abzuzweigen und mit Hilfe einer Restgas-Verdichtungsvorrichtung zur insbesondere ersten Reaktorstufe der vierstufigen Reaktoranordnung zurückzuführen. Dies wird in Verfahren 3 gemäß Figur 3 realisiert. Ein Teilstrom 25 des Restgasteilstroms 17d wird hier in einer Restgas-Verdichtungsvorrichtung 27, hier einem Recyclegas-Kompressor, auf Synthesedruck rückverdichtet und als verdichteter Teilstrom 26 des Restgasteilstroms 17d mit dem bereits verdichteten Synthesegasstrom 11 zusammengeführt.

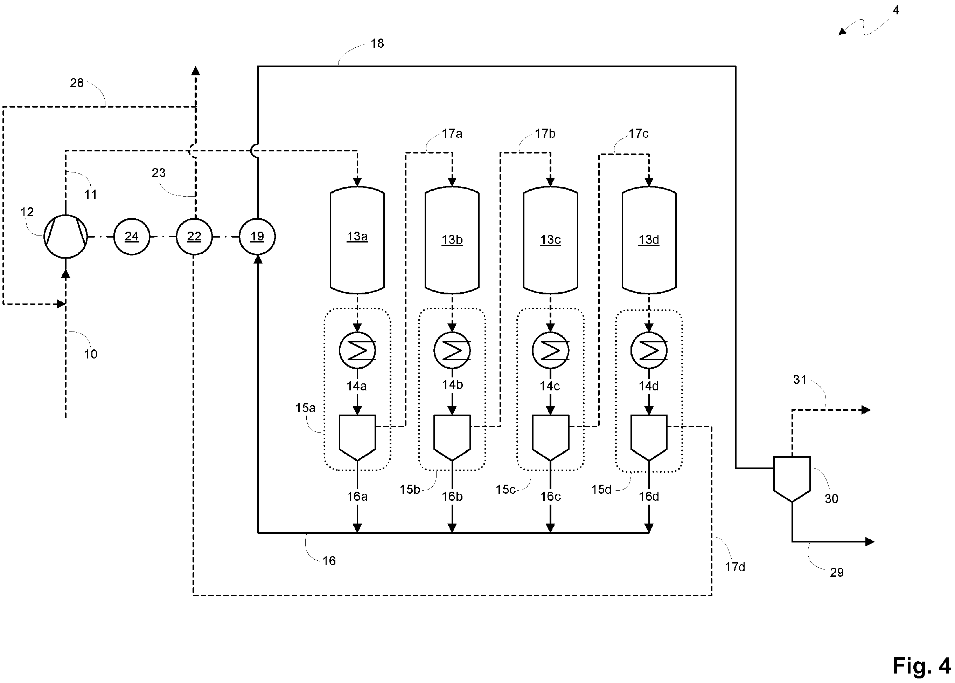

Figur 4 zeigt ein Verfahren 4 gemäß einem vierten Beispiel der Erfindung, in welchem die Synthese des Methanols analog dem Verfahren 2 in einer vierstufigen Reaktoranordnung erfolgt.

Die Verfahrensführung gemäß Figur 4 stellt eine Alternative zu Verfahren 3 bezüglich der Nutzung von im Restgasteilstrom 17d enthaltenen Wertgasen dar. Gemäß Verfahren 4 wird ein Teil des bereits druckentspannten Restgasteilstroms 23 als Teilstrom 28 abgezweigt und mit dem noch nicht verdichteten Synthesegasstrom 10 zusammengeführt. Die Verdichtung erfolgt anschließend in der Synthesegas-Verdichtungsvorrichtung 12. Die Konfiguration gemäß Figur 4 weist gegenüber der Konfiguration gemäß Figur 3 den Vorteil auf, dass keine Restgasverdichtungsvorrichtung 27 benötigt wird. Nachteilig daran ist, dass die Rückverdichtung des Teilstroms 28 einen Zusatzaufwand an elektrischer Energie in der Synthesegas-Verdichtungsvorrichtung 12 erfordert.

### Bezugszeichenliste

- 1, 2, 3, 4: Erfindungsgemäßes Verfahren
- 10: Synthesegasstrom
- 11: Verdichteter Synthesegasstrom
- 12: Synthesegas-Verdichtungsvorrichtung (Synthesegaskompressor)
- 13: Reaktorstufe
- 13a, 13b, 13c, 13d: Reaktorstufe
- 14: Produktstrom
- 14a, 14b, 14c, 14d: Produktteilstrom
- 15: Phasentrennvorrichtung
- 15a, 15b, 15c, 15d: Phasentrennvorrichtung
- 16: Rohmethanolstrom
- 16a, 16b, 16c, 16d: Rohmethanolteilstrom
- 17: Restgasstrom
- 17a, 17b, 17c, 17d: Restgasteilstrom
- 18: Druckentspannter Rohmethanolstrom
- 19: Flüssigkeit-Entspannungsvorrichtung (Entspannungsturbine)
- 20: Purge-Gasstrom
- 21: Druckentspannter Purge-Gasstrom
- 22: Gas-Entspannungsvorrichtung (Expander)
- 23: Druckentspannter Restgasteilstrom
- 24: Motor
- 25: Teilstrom des Restgasteilstroms 17d
- 26: Verdichteter Teilstrom des Restgasteilstroms 17d
- 27: Restgas-Verdichtungsvorrichtung (Recyclegas-Kompressor)
- 28: Teilstrom des druckentspannten Restgasteilstroms 23
- 29: Rohmethanolstrom
- 30: Niederdruck-Abscheider
- 31: Abgasstrom

## Patentansprüche

1. Verfahren (1,2,3,4) zur Herstellung von Methanol, aufweisend die Verfahrensschritte
a) Bereitstellen eines Synthesegasstroms (10), aufweisend zumindest ein Kohlenstoffoxid, Wasserstoff, und eine inerte Gaskomponente;
b) Verdichten des Synthesegasstroms auf Synthesedruck in einer Synthesegas-Verdichtungsvorrichtung (12);
c) Umsetzen des verdichteten Synthesegasstroms (11) an einem Methanolsynthese-Katalysator zu einem Produktstrom (14), wobei der Produktstrom (14) zumindest Methanol, Wasser, nicht umgesetztes Synthesegas und die inerte Gaskomponente aufweist, wobei das Umsetzen des verdichteten Synthesegasstroms (11) in einer Reaktoranordnung erfolgt, wobei die Reaktoranordnung zumindest eine Reaktorstufe (13) und eine Phasentrennvorrichtung (15) aufweist;
d) Auftrennen des Produktstroms (14) in der Phasentrennvorrichtung (15) in einen flüssigen Rohmethanolstrom (16), aufweisend zumindest Methanol und Wasser, sowie einen Restgasstrom (17), aufweisend zumindest nicht umgesetztes Synthesegas und die inerte Gaskomponente;
e) Entspannen zumindest eines Teils des Rohmethanolstroms (16) in einer Flüssigkeit-Entspannungsvorrichtung (19) auf einen Entspannungsdruck, welcher niedriger ist als der Synthesedruck, wobei die Flüssigkeit-Entspannungsvorrichtung (19) mechanische Arbeit verrichtet;
f) Übertragen zumindest eines Teils der von der Flüssigkeit-Entspannungsvorrichtung (19) verrichteten mechanischen Arbeit auf die Synthesegas-Verdichtungsvorrichtung (12), zum Antrieb der Synthesegas-Verdichtungsvorrichtung (12), oder
Umwandeln zumindest eines Teils der von der Flüssigkeit-Entspannungsvorrichtung (19) verrichteten mechanischen Arbeit in elektrische Energie, und Nutzung der elektrischen Energie zum Antrieb der Synthesegas-Verdichtungsvorrichtung (12).

2. Verfahren nach Anspruch 1, wobei die Reaktoranordnung eine Mehrzahl n in Serie angeordneter Reaktorstufen (13a, 13b, 13c, 13d) und eine Mehrzahl p Phasentrennvorrichtungen (15a, 15b, 15c, 15d) aufweist, wobei jeder der Reaktorstufen eine Phasentrennvorrichtung zugeordnet ist, wobei die Phasentrennvorrichtung jeweils stromabwärts zur zugeordneten Reaktorstufe angeordnet ist, und in jeder der Phasentrennvorrichtungen (15a, 15b, 15c, 15d) ein flüssiger Rohmethanolteilstrom (14a, 14b, 14c, 14d) und ein Restgasteilstrom (17a, 17b, 17c, 17d) erzeugt wird, und wobei der in einer Phasentrennvorrichtung erzeugte Restgasteilstrom zumindest teilweise in die jeweils nachfolgend angeordnete Reaktorstufe eingeschleust wird, und wobei der in der letzten Phasentrennvorrichtung (15d) erzeugte Restgasteilstrom (17d) aus der Reaktoranordnung ausgeschleust wird.

3. Verfahren nach Anspruch 2, aufweisend die Verfahrensschritte
g) Entspannen zumindest eines Teils des aus der Reaktoranordnung ausgeschleusten Restgasteilstroms (17d) in einer Gas-Entspannungsvorrichtung (22) auf einen Entspannungsdruck, welcher niedriger ist als der Synthesedruck, wobei die Gas-Entspannungsvorrichtung (22) mechanische Arbeit verrichtet;
h) Übertragen zumindest eines Teils der von der Gas-Entspannungsvorrichtung (22) verrichteten mechanischen Arbeit auf die Synthesegas-Verdichtungsvorrichtung (12), zum Antrieb der Synthesegas-Verdichtungsvorrichtung (12), oder
Umwandeln zumindest eines Teils der von der Gas-Entspannungsvorrichtung (22) verrichteten mechanischen Arbeit in elektrische Energie, und Nutzung der elektrischen Energie zum Antrieb der Synthesegas-Verdichtungsvorrichtung (12).

4. Verfahren nach Anspruch 3, wobei der gesamte aus der Reaktoranordnung ausgeschleuste Restgasteilstrom (17d) in der Gas-Entspannungsvorrichtung entspannt (22) wird.

5. Verfahren nach einem der Ansprüche 3 oder 4, wobei ein Anteil des aus der Reaktoranordnung ausgeschleusten Restgasteilstroms (17d) in der Gas-Entspannungsvorrichtung (22) entspannt wird, und ein weiterer Anteil (25) des aus der Reaktoranordnung ausgeschleusten Restgasteilstroms in einer Restgas-Verdichtungsvorrichtung (27) auf Synthesedruck verdichtet wird, und der verdichtete Restgasteilstrom (26) zumindest zu einer der Mehrzahl in Serie angeordneter Reaktorstufen (13a, 13b, 13c, 13d) zurückgeführt wird, vorzugsweise zur ersten der Mehrzahl in Serie angeordneter Reaktorstufen zurückgeführt wird.

6. Verfahren nach einem der Ansprüche 3 bis 5, wobei zumindest ein Teil (28) des auf Entspannungsdruck entspannten Restgasteilstroms (23) in der Synthesegas-Verdichtungsvorrichtung (12) auf Synthesedruck rückverdichtet wird, und der verdichtete Restgasteilstrom der Reaktoranordnung zugeführt wird.

7. Verfahren nach einem der vorherigen Ansprüche, wobei der Anteil der inerten Gaskomponente im Synthesegasstrom zumindest 1 Vol.-% beträgt, oder zumindest 5 Vol.-% beträgt, oder zumindest 10 Vol.-% beträgt, oder zumindest 20 Vol.-% beträgt.

8. Verfahren nach einem der vorherigen Ansprüche, wobei die inerte Gaskomponente Stickstoff und/oder Methan aufweist.

9. Verfahren nach einem der vorherigen Ansprüche, wobei der Synthesedruck mindestens 70 bar beträgt, oder mindestens 75 bar beträgt, oder mindestens 80 bar beträgt, oder mindestens 85 bar beträgt.

10. Verfahren nach einem der vorherigen Ansprüche, wobei der Rohmethanolstrom in der Flüssigkeit-Entspannungsvorrichtung (19) auf einen Druck von 1 bar bis 3 bar entspannt wird.

11. Verfahren nach einem der vorherigen Ansprüche, wobei der Rohmethanolstrom (16) einer stromabwärts angeordneten thermischen Trennvorrichtung zur Trennung des Rohmethanols in Methanol und Wasser zugeführt wird, wobei die thermische Trennvorrichtung bei einem vorgegebenen Druck betrieben wird, und der Rohmethanolstrom (16) in der Flüssigkeit-Entspannungsvorrichtung auf einen Druck entspannt wird, welcher dem vorgegebenen Druck in der thermischen Trennvorrichtung entspricht.

12. Verfahren nach einem der Ansprüche 2 bis 11, wobei der Restgasteilstrom (17d) in der Gas-Entspannungsvorrichtung (22) auf einen Druck von 1 bar bis 3 bar entspannt wird.

13. Verfahren nach einem der Ansprüche 2 bis 12, wobei für die Mehrzahl n in Serie angeordneter Reaktorstufen (13a, 13b, 13c, 13d) und die Mehrzahl p Phasentrennvorrichtungen (15a, 15b, 15c, 15d) gilt
- n = 3 bis 6, vorzugsweise n = 4, sowie
- p = 3 bis 6, vorzugsweise p = 4, und wobei gilt
- n = p.

## Claims

1. A method (1,2,3,4) for producing methanol, comprising the method steps
a) Providing a synthesis gas stream (10), comprising at least one carbon oxide, hydrogen, and an inert gas component;
b) Compressing the synthesis gas stream to a synthesis pressure in a synthesis gas compression device (12);
c) Reacting the compressed synthesis gas stream (11) on a methanol synthesis catalyst to form a product stream (14), wherein the product stream (14) comprises at least methanol, water, unreacted synthesis gas and the inert gas component, wherein the reaction of the compressed synthesis gas stream (11) takes place in a reactor arrangement, wherein the reactor arrangement comprises at least one reactor stage (13) and a phase separation device (15);
d) Separating the product stream (14) in the phase separation device (15) into a liquid crude methanol stream (16), comprising at least methanol and water, and a residual gas stream (17), comprising at least unreacted synthesis gas and the inert gas component;
e) Expanding at least a part of the crude methanol stream (16) in a liquid expansion device (19) to an expansion pressure which is lower than the synthesis pressure, wherein the liquid expansion device (19) performs mechanical work;
f) Transferring at least a part of the mechanical work performed by the liquid expansion device (19) to the synthesis gas compression device (12), for driving the synthesis gas compression device (12), or
Converting at least a part of the mechanical work performed by the liquid expansion device (19) into electrical energy, and using the electrical energy to drive the synthesis gas compression device (12).

2. The method according to claim 1, wherein the reactor arrangement comprises a plurality n of series-connected reactor stages (13a, 13b, 13c, 13d) and a plurality p of phase separation devices (15a, 15b, 15c, 15d), wherein each of the reactor stages is assigned a phase separation device, wherein the phase separation device is arranged downstream of the assigned reactor stage in each case, and in each of the phase separation devices (15a, 15b, 15c, 15d) a liquid crude methanol partial stream (14a, 14b, 14c, 14d) and a residual gas partial stream (17a, 17b, 17c, 17d) is produced, and wherein the residual gas partial stream produced in a phase separation device is at least partially fed into the respectively subsequent reactor stage, and wherein the residual gas partial stream (17d) produced in the last phase separation device (15d) is discharged from the reactor arrangement.

3. The method according to claim 2, comprising the method steps
g) Expanding at least a part of the residual gas partial stream (17d) discharged from the reactor arrangement in a gas expansion device (22) to an expansion pressure which is lower than the synthesis pressure, wherein the gas expansion device (22) performs mechanical work;
h) Transferring at least a part of the mechanical work performed by the gas expansion device (22) to the synthesis gas compression device (12), for driving the synthesis gas compression device (12), or
Converting at least a part of the mechanical work performed by the gas expansion device (22) into electrical energy, and using the electrical energy to drive the synthesis gas compression device (12).

4. The method according to claim 3, wherein the entire residual gas partial stream (17d) discharged from the reactor arrangement is expanded (22) in the gas expansion device.

5. The method according to one of claims 3 or 4, wherein a portion of the residual gas partial stream (17d) discharged from the reactor arrangement is expanded in the gas expansion device (22), and a further portion (25) of the residual gas partial stream discharged from the reactor arrangement is compressed to synthesis pressure in a residual gas compression device (27), and the compressed residual gas partial stream (26) is recycled to at least one of the plurality of series-connected reactor stages (13a, 13b, 13c, 13d), preferably to the first of the plurality of series-connected reactor stages.

6. The method according to one of claims 3 to 5, wherein at least a part (28) of the residual gas partial stream (23) expanded to the expansion pressure is recompressed to synthesis pressure in the synthesis gas compression device (12), and the compressed residual gas partial stream is fed to the reactor arrangement.

7. The method according to one of the preceding claims, wherein the proportion of the inert gas component in the synthesis gas stream is at least 1% by volume, or at least 5% by volume, or at least 10% by volume, or at least 20% by volume.

8. The method according to one of the preceding claims, wherein the inert gas component comprises nitrogen and/or methane.

9. The method according to one of the preceding claims, wherein the synthesis pressure is at least 70 bar, or at least 75 bar, or at least 80 bar, or at least 85 bar.

10. The method according to one of the preceding claims, wherein the crude methanol stream is expanded in the liquid expansion device (19) to a pressure of 1 bar to 3 bar.

11. The method according to one of the preceding claims, wherein the crude methanol stream (16) is fed to a downstream-arranged thermal separation device for separating the crude methanol into methanol and water, wherein the thermal separation device is operated at a given pressure, and the crude methanol stream (16) is expanded in the liquid expansion device to a pressure which corresponds to the given pressure in the thermal separation device.

12. The method according to one of claims 2 to 11, wherein the residual gas partial stream (17d) is expanded in the gas expansion device (22) to a pressure of 1 bar to 3 bar.

13. The method according to one of claims 2 to 12, wherein for the plurality n of series-connected reactor stages (13a, 13b, 13c, 13d) and the plurality p of phase separation devices (15a, 15b, 15c, 15d) the following applies
- n = 3 to 6, preferably n = 4, and
- p = 3 to 6, preferably p = 4, and wherein it holds that
- n = p.

## Revendications

1. Procédé (1,2,3,4) de production de méthanol, comprenant les étapes de procédé suivantes :
a) la fourniture d'un courant de gaz de synthèse (10), comprenant au moins un oxyde de carbone, de l'hydrogène, et un composant gazeux inerte ;
b) la compression du courant de gaz de synthèse à une pression de synthèse dans un dispositif de compression de gaz de synthèse (12) ;
c) la réaction du courant de gaz de synthèse comprimé (11) sur un catalyseur de synthèse de méthanol pour former un courant de produit (14), dans lequel le courant de produit (14) comprend au moins du méthanol, de l'eau, du gaz de synthèse n'ayant pas réagi et le composant gazeux inerte, dans lequel la réaction du courant de gaz de synthèse comprimé (11) s'effectue dans un agencement de réacteur, dans lequel l'agencement de réacteur comprend au moins un étage de réacteur (13) et un dispositif de séparation de phases (15) ;
d) la séparation du courant de produit (14) dans le dispositif de séparation de phases (15) en un courant de méthanol brut liquide (16), comprenant au moins du méthanol et de l'eau, ainsi qu'un courant de gaz résiduel (17), comprenant au moins du gaz de synthèse n'ayant pas réagi et le composant gazeux inerte ;
e) la détente d'au moins une partie du courant de méthanol brut (16) dans un dispositif de détente de liquide (19) à une pression de détente qui est inférieure à la pression de synthèse, dans lequel le dispositif de détente de liquide (19) effectue un travail mécanique ;
f) le transfert d'au moins une partie du travail mécanique effectué par le dispositif de détente de liquide (19) au dispositif de compression de gaz de synthèse (12), pour entraîner le dispositif de compression de gaz de synthèse (12), ou
la conversion d'au moins une partie du travail mécanique effectué par le dispositif de détente de liquide (19) en énergie électrique, et l'utilisation de l'énergie électrique pour entraîner le dispositif de compression de gaz de synthèse (12).

2. Procédé selon la revendication 1, dans lequel l'agencement de réacteur comprend une pluralité n d'étages de réacteur (13a, 13b, 13c, 13d) agencés en série et une pluralité p de dispositifs de séparation de phases (15a, 15b, 15c, 15d), dans lequel à chacun des étages de réacteur est associé un dispositif de séparation de phases, le dispositif de séparation de phases étant à chaque fois agencé en aval de l'étage de réacteur associé, et dans chacun des dispositifs de séparation de phases (15a, 15b, 15c, 15d), un courant partiel de méthanol brut liquide (14a, 14b, 14c, 14d) et un courant partiel de gaz résiduel (17a, 17b, 17c, 17d) sont produits, et dans lequel le courant partiel de gaz résiduel produit dans un dispositif de séparation de phases est au moins partiellement introduit dans l'étage de réacteur respectivement suivant, et dans lequel le courant partiel de gaz résiduel (17d) produit dans le dernier dispositif de séparation de phases (15d) est évacué de l'agencement de réacteur.

3. Procédé selon la revendication 2, comprenant les étapes de procédé suivantes :
g) la détente d'au moins une partie du courant partiel de gaz résiduel (17d) évacué de l'agencement de réacteur dans un dispositif de détente de gaz (22) à une pression de détente qui est inférieure à la pression de synthèse, dans lequel le dispositif de détente de gaz (22) effectue un travail mécanique ;
h) le transfert d'au moins une partie du travail mécanique effectué par le dispositif de détente de gaz (22) au dispositif de compression de gaz de synthèse (12), pour entraîner le dispositif de compression de gaz de synthèse (12), ou
la conversion d'au moins une partie du travail mécanique effectué par le dispositif de détente de gaz (22) en énergie électrique, et l'utilisation de l'énergie électrique pour entraîner le dispositif de compression de gaz de synthèse (12).

4. Procédé selon la revendication 3, dans lequel la totalité du courant partiel de gaz résiduel (17d) évacué de l'agencement de réacteur est détendue (22) dans le dispositif de détente de gaz.

5. Procédé selon l'une des revendications 3 ou 4, dans lequel une partie du courant partiel de gaz résiduel (17d) évacué de l'agencement de réacteur est détendue dans le dispositif de détente de gaz (22), et une autre partie (25) du courant partiel de gaz résiduel évacué de l'agencement de réacteur est comprimée à la pression de synthèse dans un dispositif de compression de gaz résiduel (27), et le courant partiel de gaz résiduel comprimé (26) est recyclé vers au moins l'un de la pluralité d'étages de réacteur agencés en série (13a, 13b, 13c, 13d), de préférence vers le premier de la pluralité d'étages de réacteur agencés en série.

6. Procédé selon l'une des revendications 3 à 5, dans lequel au moins une partie (28) du courant partiel de gaz résiduel (23) détendu à la pression de détente est recomprimée à la pression de synthèse dans le dispositif de compression de gaz de synthèse (12), et le courant partiel de gaz résiduel comprimé est amené à l'agencement de réacteur.

7. Procédé selon l'une des revendications précédentes, dans lequel la proportion du composant gazeux inerte dans le courant de gaz de synthèse est d'au moins 1 % en volume, ou d'au moins 5 % en volume, ou d'au moins 10 % en volume, ou d'au moins 20 % en volume.

8. Procédé selon l'une des revendications précédentes, dans lequel le composant gazeux inerte comprend de l'azote et/ou du méthane.

9. Procédé selon l'une des revendications précédentes, dans lequel la pression de synthèse est d'au moins 70 bar, ou d'au moins 75 bar, ou d'au moins 80 bar, ou d'au moins 85 bar.

10. Procédé selon l'une des revendications précédentes, dans lequel le courant de méthanol brut est détendu dans le dispositif de détente de liquide (19) à une pression de 1 bar à 3 bar.

11. Procédé selon l'une des revendications précédentes, dans lequel le courant de méthanol brut (16) est amené à un dispositif de séparation thermique agencé en aval pour la séparation du méthanol brut en méthanol et en eau, dans lequel le dispositif de séparation thermique est exploité à une pression prédéfinie, et le courant de méthanol brut (16) est détendu dans le dispositif de détente de liquide à une pression qui correspond à la pression prédéfinie dans le dispositif de séparation thermique.

12. Procédé selon l'une des revendications 2 à 11, dans lequel le courant partiel de gaz résiduel (17d) est détendu dans le dispositif de détente de gaz (22) à une pression de 1 bar à 3 bar.

13. Procédé selon l'une des revendications 2 à 12, dans lequel pour la pluralité n d'étages de réacteur agencés en série (13a, 13b, 13c, 13d) et la pluralité p de dispositifs de séparation de phases (15a, 15b, 15c, 15d) s'applique ce qui suit
- n = 3 à 6, de préférence n = 4, ainsi que
- p = 3 à 6, de préférence p = 4, et dans lequel il est vrai que
- n = p.
